(19) Europäisches Patentamt
European Patent Office
Office européen des brevets

(11) Publication number: **0 448 188 A2**

(12) **EUROPEAN PATENT APPLICATION**

(21) Application number: **91250066.7**

(22) Date of filing: **12.03.91**

(51) Int. Cl.[5]: **C07D 513/04**, C07C 233/15, C07C 233/25, C07C 331/08, //(C07D513/04,285:00,209:00)

(30) Priority: **15.03.90 DE 4008691**
**29.09.90 DE 4031188**

(43) Date of publication of application:
**25.09.91 Bulletin 91/39**

(84) Designated Contracting States:
**AT BE CH DE DK ES FR GB GR IT LI LU NL SE**

(71) Applicant: **SCHERING AKTIENGESELLSCHAFT**
**Müllerstrasse 170/178**
**W-1000 Berlin 65(DE)**

(72) Inventor: **Ganzer, Michael, Dr.**
**Eichborndamm 279**
**W-1000 Berlin 26(DE)**
Inventor: **Dorfmeister, Gabriele, Dr.**
**Heiligenseestrasse 70**
**W-1000 Berlin 27(DE)**
Inventor: **Franke, Wilfried, Dr.**
**Spiessergasse 6b**
**W-1000 Berlin 27(DE)**
Inventor: **Seba, Hartmut, Dr.**
**Bergstrasse 4**
**W-3303 Vechelde(DE)**

(54) **Process and intermediates for the preparation of substituted 6-(3,5,6,7-tetrahydropyrrolo-[2,1-c][1,2,4]-thiadiazol-3-ylidenimino)-7-fluoro-2H-1,4-benzoxazin-3(4H)-ones.**

(57) The invention relates a new process for the preparation of substituted 6-(3,5,6,7-tetrahydropyrrolo[2,1-c]-[1,2,4]-thiadiazol-3-ylidenimino)-7-fluoro-2H-1,4-benzoxazin-3(4H)-ones of general formula I

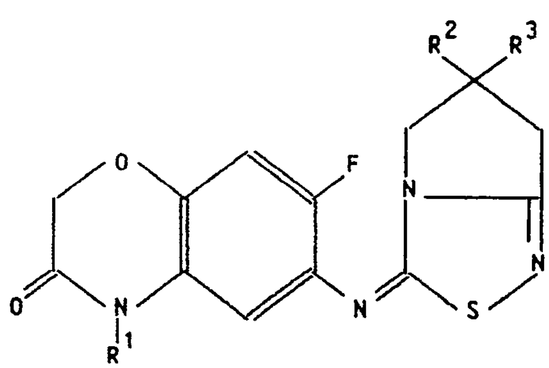

(I)

in which
$R^1$ is $C_1$-$C_6$-alkyl, $C_2$-$C_6$-alkenyl or $C_3$-$C_6$-alkynyl, and
$R^2$ and $R^3$, which may be the same or different, are hydrogen or $C_1$-$C_4$-alkyl,

EP 0 448 188 A2

which starts from the known
a 2-amino-5-fluorophenol of formula II

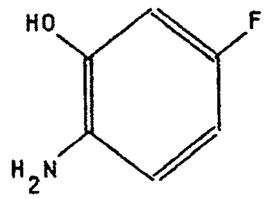

(II)

via the new acetanilide of formula IV

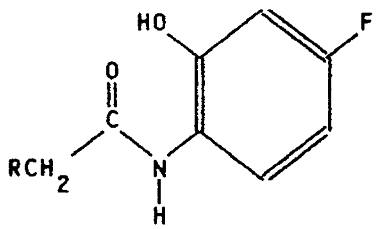

(IV)

in which R is hydrogen or chlorine,
via the new 5-nitroacetanilide of formula V

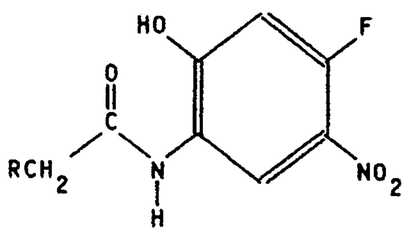

(V) ,

via the new 5-aminoacetanilide of formula VI

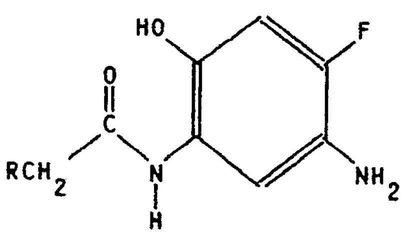

(VI) ,

via the new 5-isothiocyanatoacetanilide of formula VIII

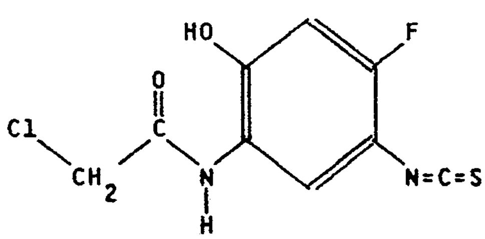

(VIII) ,

via the known benzoxazinone isothiocyanate of general formula IX

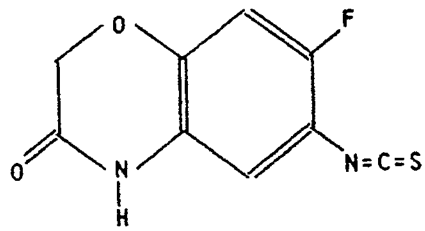

(IX)

and via the known iminothiadiazole of general formula XI

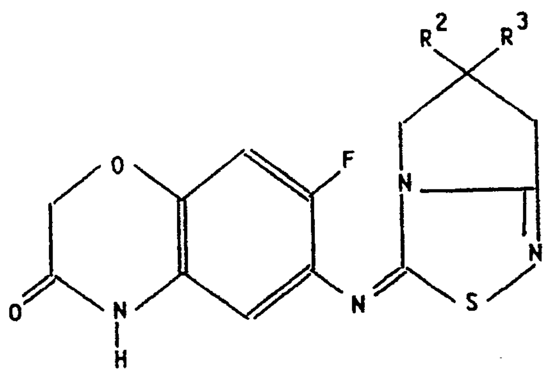

(XI)

This invention relates to a new process and new intermediates for the preparation of substituted 6-(3,5,6,7-tetrahydropyrrolo[2,1-c][1,2,4]thiadiazol-3-ylidenimino)-7-fluoro-2H-1,4-benzoxazin-3(4H)-ones of general formula I

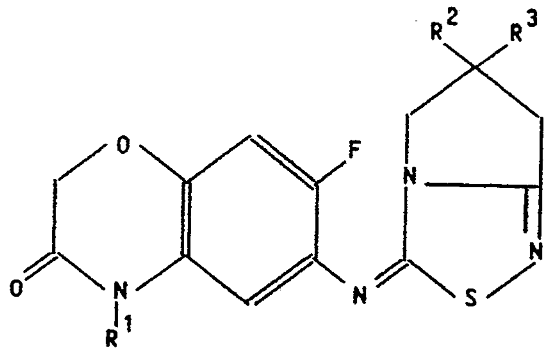

(I) ,

in which
$R^1$ is $C_1$-$C_6$-alkyl, $C_2$-$C_6$-alkenyl or $C_3$-$C_6$-alkynyl, and
$R^2$ and $R^3$ which may be the same or different are hydrogen or $C_1$-$C_4$-alkyl.

These compounds have excellent herbicidal activity against a broad spectrum of monocotyledonous and dicotyledonous weeds in agricultural crops. Their preparation and use are described in EP 311 135.

The object of the present invention is to provide a new process which allows preparation of compounds of general formula I without any problems, and under mild reaction conditions.

This object is solved by a process for the preparation of 6-(3,5,6,7-tetrahydropyrrolo[2,1-c][1,2,4]-thiadiazol-3-ylidenimino)-7-fluoro-2H-1,4-benzoxazin-3(4H)-ones in which

a) a 2-amino-5-fluorophenol of formula II

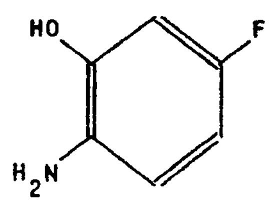

(II)

is reacted with chloracetyl chloride, acetyl chlride or acetic anhydride, in the presence of an acid binding agent, in a suitable solvent.
b) the acetanilide, so formed, of formula IV

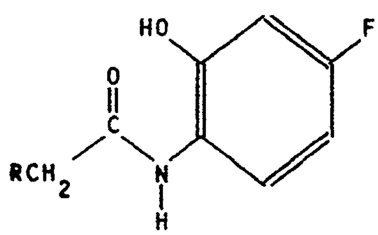

(IV)

in which R is hydrogen or chlorine, is nitrated with nitric acid, an inorganic or organic derivative thereof, optionally in a suitable solvent,
c) the 5-nitroacetanilide, so formed, of formula V

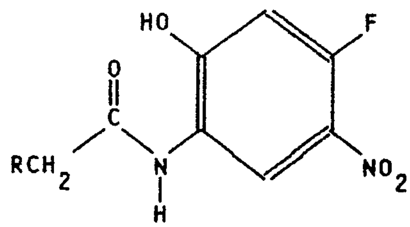

(V)

is hydrogenated with hydrogen, in the presence of a suitable catalyst in an inert solvent, or reduced with a chemical reducing agent in the presence of an inert solvent,

d) the 5-aminoacetanilide, so formed, of formula VI

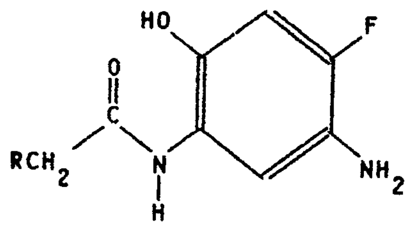

(VI)

is treated with a compound of formula VII

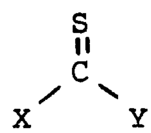

(VII)

in which X is chloro or imidazole and Y is chloro, imidazole or dialkylamino, in a suitable inert solvent, optionally with the addition of an acid binding agent,

e) the 5-isothiocyanatoacetanilide, so formed, of formula VIII

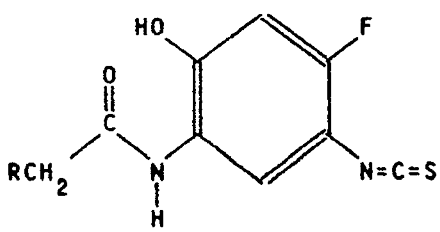

(VIII)

is cyclised in the presence of catalytic to equimolar amounts of an inorganic or organic base,

f) the benzoxazinone isothiocyanate, so formed, of general formula IX

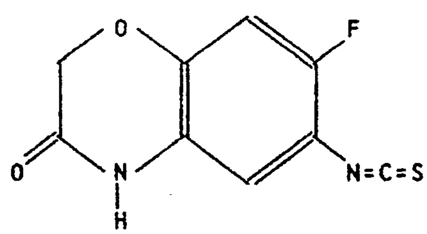

(IX)

is reacted with a compound of general formula X

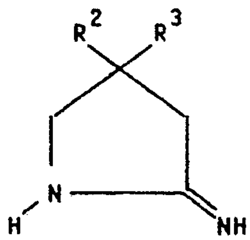

(X) ,

in which $R^2$ and $R^3$ have the meanings given in general the formula I, in an inert solvent, and the resulting compound cyclised in the presence of an oxidising agentb to the iminothiadiazole ring, and finally

g) the iminothiadiazole, so formed, of general formula XI

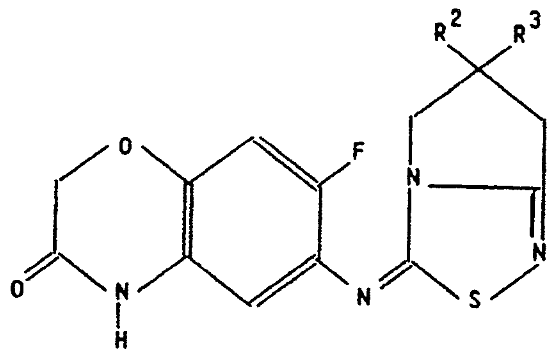

(XI) ,

in which $R^2$ and $R^3$ have the meanings given in general the formula I, is reacted with a compound of general formula XII

$R^1$-Z (XII)

in which $R^1$ has the meaning given in general formula I and Z is chlorine, bromine, iodine, p-toluenesulphonyloxy or methanesulphonyloxy, optionally with the addition of a base in a suitable solvent.

Process step a) is generally carried out by reacting the starting materials in a suitable solvent, optionally with the addition of inorganic or organic bases at a temperature between 0 and 150°C. The reaction can optionally be carried out in a two phase system, for example water-methyl isobutyl ketone.

Suitable bases are alkali and alkaline earth metal hydroxides, carbonates and/or hydrogen carbonates, tertiary aliphatic and aromatic amines as well as heterocyclic bases. Examples include sodium and potassium hydroxide, sodium and potassium carbonate, sodium and potassium hydrogen carbonate, triethylamine and pyridine.

Suitable solvents include hydrocarbons such as toluene, chlorinated hydrocarbons such as methylene chloride or chloroform, ethers, such as diethyl ether or tetrahydrofuran, ketones, such as acetone, butanone or methyl isobutyl ketone, and nitrile, such as acetonitrile, and also two phase mixtures with water.

The resulting N-(4-fluoro-2-hydroxyphenyl-2-chloracetamide is new and is also within the scope of this invention.

Process step b) is generally carried out according to known processes for nitration of a phenol, as described for example in Houben-Weyl, Vol. X/1, page 559 ff.

For example nitration of the aromatic compound has been carried out in sulphuric acid solution with the addition of sulphuric acid/nitric acid mixtures with ice cooling.

The resulting N-(4-fluoro-2-hydroxy-5-nitrophenyl)-2-acetamide and N-(4-fluoro-2-hydroxy-5-nitrophenyl)-2-chloracetamide are new and also are within the scope of the invention.

The process step c) is generally carried out according to known processes for a reduction of an aromatic nitro group, for example as described in Houben-Weyl, Vol. XI/1, page 360 ff.

For example reduction with iron powder in dilute acetic acid in the presence of acetic acid and ethyl acetate has been carried out.

The resulting N-(4-fluoro-2-hydroxy-5-hydroxyphenyl)-2-acetamide and N-(5-amino-4-fluoro-2-hydroxyphenyl)-2-chloracetamide are new and also fall within the scope of the invention.

The process step d) is generally carried out by reacting the starting materials in a suitable solvent, optionally with the addition of inorganic or organic bases, at a temperature between 0 and 50 ° C. The reaction can optionally be also carried out in a two phase mixture, optionally with the addition of a phase transfer catalyst.

Suitable bases include alkali and alkaline earth metal hydroxides carbonates and/or hydrogen carbonates, as well as tertiary aliphatic and aromatic amines. Examples include sodium and potassium hydroxide, sodium and potassium carbonate, calcium carbonate, sodium potassium hydrogen carbonate and triethylamine.

Suitable solvents include hydrocarbons, such as toluene, chlorinated hydrocarbons, such as methylene chloride or 1,2-dichloroethane, ketones, such as acetone or butanone and also water.

The resulting N-(4-fluoro-2-hydroxy-5-isothiocyanatophenyl)-2-acetamide and N-(4-fluoro-2-hydroxy-5-isothiocyanatophenyl)-2-chloracetamide are new and also fall within the scope of the invention.

It is already known that conversion of anilines to the corresponding isothiocyanates sometimes occurs without affecting further functional groups (Houben-Weyl, Vol E 4, fourth edition 1983, page 838). On the other hand, for example, 4-hydroxyphenyl isothiocyanate cannot be obtained by reacting 4-hydroxyaniline with thiophosgene (Coll. Czech. Chem. Commun. 30 (1965) 3658, see also "The Chemistry of the -NCS Group" in Patai "The Chemistry of Cyanates and Their Derivatives" Part 2 (1977), John Wiley & Sons Ltd).

Should the second reactive group be in a position suitable for ring closure, such as in 1,2-diaminobenzene or 2-aminophenol, then this leads to the formation of a corresponding sulphur containing heterocycles, benzimidazolethione or benzoxazolethione (Ber. Dtsch. Chem. Ges. 58 (1925) 2151 and J. Soc. Chem. Ind. 45 (1926) 81).

Therefore, it is surprising that the conversion of N-(5-amino-4-fluoro-2-hydroxyphenyl)-2-chloroacetamide or the corresponding 2-acetamide leads easily and in good yield to the corresponding isothiocyanate, without formation of the corresponding benzoxazolethione.

Process step e) is generally carried out by cyclising the starting material in a suitable solvent, optionally with the addition of an inorganic or organic base at a temperature between 0 and 150 ° C.

Suitable bases include alkali and alkaline earth metal hydroxides alcoholates, carbonates and hydrogen carbonates, tertiary aliphatic and aromatic amines, as well as heterocyclic bases. Examples are sodium and potassium hydroxide, sodium tert.-butanolate, sodium and potassium carbonate, sodium and potassium hydrogen carbonate, triethylamine and pyridine.

Suitable solvents include hydrocarbons, such as toluene, chlorinated hydrocarbons, such as methylene chloride or chloroform, ethers, such as diisopropyl ether or tetrahydrofuran, alcohols, such as tert.-butanol, ketones, such as acetone or methyl isobutylketone and also nitriles, such as acetonitrile.

Process step f) is generally carried out by reacting the reaction materials in a suitable solvent, such as for example ether, methylene chloride, chloroform or ethyl acetate, at a temperature between -50 ° C and +50 ° C, optionally with the addition of an inorganic or organic base. It can also be carried out in a two phase system, optionally with the use of a phase transfer catalyst.

The immediate resulting compound of general formula XIII

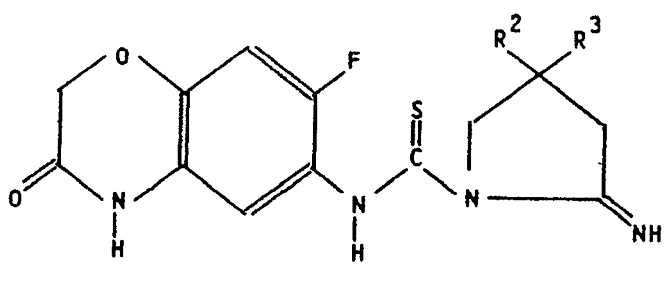

(XIII)

in which $R^2$ and $R^3$ have the meaning given in general formula I is thermally unstable and is thus treated preferably without isolation in the next reaction.

The ring formation is carried out using an oxidising agent in an inert organic solvent. It can also be carried out in a two phase system with water optionally using a phased transfer catalyst.

Solvents include halogenated hydrocarbons, such as methylene chloride, chloroform or chlorobenzene, amides, such as N,N-dimethylformamide, and esters, such as ethyl acetate.

The condensation reaction is carried out under ring formation conditions in the presence of active acid acceptors generally used with oxidising agents.

Suitable acid acceptors include organic bases, such as triethylamine, pyridine or dimethylamine, as well

as inorganic bases, such as sodium hydroxide, potassium carbonate or calcium carbonate.

Oxidising agents can be for example bromine, chlorine or sodium hypochlorite.

The process step g) is generally carried out by reacting the starting materials in a suitable solvent optionally with the addition of inorganic or organic bases at a temperature between -10° C and 150° C. The reaction can also be carried out in a two phase system with water with the addition of phase transfer catalyst.

Suitable bases can be alkali and alkaline earth metal hydroxides, alcoholates and carbonates and alkali metal hydrides. Examples include sodium and potassium hydroxide, sodium potassium tert.-butanolate, sodium and potassium carbonate as well as sodium hydride and butyllithium.

Suitable solvents include hydrocarbons, such as toluene, chlorinated hydrocarbons, such as methylene chloride or chloroform, ethers, such as diispropyl ether or tetrahydrofuran, alcohols, such as methanol or ethanol, ketones, such as acetone or butanone, amides, such as dimethylformamide, sulphoxides, such as dimethyl sulphoxide and also nitriles, such as acetonitrile.

The following examples illustrate the invention.

Example 1.1 - Process step a)

N-(4-Fluoro-2-hydroxyphenyl)-2-chloracetamide

12.68 g Chloroacetylchloride was added slowly, dropwise, to a two-phase system consisting of 60 ml water and 12.6 g sodium hydrogen carbonate as well as 60 ml methyl isobutyl ketone and 12.7 g 2-amino-5-fluorophenol at 0° C. The mixture was stirred for 3 hours at room temperature, the phases separated and the organic phase dried over magnesium sulphate and heated for a short time with active charcoal. The mixture was filtered, the methyl isobutyl ketone removed in vacuo and the residue washed with hexane.

Yield: 19.6 g = 96% of theory
Mp: 145-146° C

Example 1.2 - Process step a)

N-(4-Fluoro-2-hydroxyphenyl)acetamide

84 g Acetic anhydride was added dropwise to a solution of 97 g 5-fluoro-2-aminophenol in 500 ml ethanol. The mixture was stirred for one hour at room temperature after which some of the product crystallises out. The crystals were filtered off and the residue concentrated in vacuo. The residue was washed with hexane and dried in vacuo.

Yield: 115g = 89% of theory
Mp: 174 - 176° C

Example 2.1 - Process step b)

N-(4-Fluoro-2-hydroxy-5-nitrophenyl)-2-chloracetamide

A cooled solution of 200 ml concentrated sulphuric acid and 18.9 g of 100% nitric acid was added dropwise at 0° C to a solution of 61 g N-(4-fluoro-2-hydroxyphenyl)-2-chloracetamide in 500 ml concentrated sulphuric acid. The mixture was stirred for 30 minutes at room temperature and poured into 2 litres of ice-water. The precipitate was filtered off, washed with water and dried in vacuo.

Yield: 55.8 g = 75% of theory
Mp: 218° C

Example 2.2 - Process step b)

N-(4-Fluoro-2-hydroxy-5-nitrophenyl)acetamide

A cooled solution of 27 g 65% nitric acid and 27 g concentrated sulphuric acid was added to a solution of 45 g N-(4-fluoro-2-hydroxyphenyl)-acetamide in 350 ml concentrated sulphuric acid, cooled to -12° C. The mixture was stirred for 15 minutes at -10° C and poured into 400 g ice-water. The precipitate was filtered, washed with water and dried in vacuo.

Yield: 45 g = 72% of theory

Mp: 257° C

Example 3.1 - Process step c)

N-(5-Amino-4-fluoro-2-hydroxyphenyl)-2-chloracetamide

A solution of 29.8 g N-(4-fluoro-2-hydroxy-5-nitrophenyl)-2-chloracetamide in 1.3 litres ethyl acetate and 300 ml acetic acid was added dropwise to a suspension of 27.9 g iron powder in 100 ml 5% acetic acid heated to 80° C. The mixture was heated under reflux for one hour, cooled and poured into 1.5 litre ice-water. The iron salt was filtered off, the organic phase separated, washed with water, dried over magnesium sulphate and the solvent removed in vacuo. The solid residue was washed three times with hexane and dried in vacuo.

Yield: 22.3 g = 85% of theory
Mp: 123-124° C

Example 3.2 - Process step c)

N-(5-Amino-4-fluoro-2-hydroxyphenyl)acetamide

3 g Palladium-charcoal was added to a solution of 44 g N-(4-fluoro-2-hydroxy-5-nitrophenyl)acetamide in 100 ml ethanol. The mixture was hydrogenated at room temperature with hydrogen. After 3 hours the reaction was finished. The catalyst was filtered off and solvent removed in vacuo. The product was used in the next stage without further purification.

Yield: 35.5 g = 94% of theory
Mp: 165° C

Example 4.1 - Process step d)

N-(4-Fluoro-2-hydroxy-5-isothiocyanatophenyl)-2-chloracetamide

3.45 g Thiophosgene was added dropwise, with stirring, to a two phase mixture of 6.56 g N-(5-amino-fluoro-2-hydroxyphenyl)-2-chloracetamide in 200 ml ethyl acetate and 3 g calcium carbonate in 50 ml water at a temperature of 0-5° C. The mixture was stirred for one hour at room temperature, the phases separated, the organic phases dried over magnesium sulphate and the solvent removed in vacuo. The residue was recrystallised from a small amount of ethyl acetate.

Yield: 4.7 g = 60% of theory
Mp: 207° C (dec.)

Example 4.2 - Process step d)

N-(Fluoro-2-hydroxy-5-isothiocyanatophenyl)acetamide

39.6 g Sodium hydrogen carbonate was added to a solution of 34 g N-(5-amino-4-fluoro-2-hydroxyphenyl)acetamide in 1 litre ethanol, cooled to 0° C, and 27.1 g thiophosgene added, dropwise. The mixture was stirred for one hour at room temperature. The product party crystallised out. The mixture was filtered and the mother liquor concentrated. The residue was recrystallised from a small amount of ethyl acetate.

Yield: 39.7 g = 95% of theory
Mp: 222-224° C

Example 5.1 - Process step e)

7-Fluoro-6-isothiocyanato-2H-1,4-benzoxazine-3(4H)-one

A mixture of 1.3 g N-(4-fluoro-2-hydroxy-5-isothiocyanatophenyl)-2-chloracetamide in 50 ml acetone and 0.6 g powdered potassium carbonate was heated under reflux for 3 hours. The solvent was removed in vacuo and the residue digested in water. The crystals were removed and recrystallised from a small amount of ethyl acetate.

Yield: 0.6 g = 54% of theory

Mp: 244-246° C

Example 5.2 - Process Step e)

7-Fluoro-6-isothiocyanato-2H-1,4-benzoxazin-3(4H)-one

3 g Potassium carbonate was added to a solution of 2 g N-(4-fluoro-2-hydroxy-5-isothiocyanatophenyl)-acetamide in 100 ml methyl isobutyl ketone. 1.24 g Chloroacetyl chloride was added dropwise at room temperature. The mixture was heated for one hour at 120° C, 0.8g potassium carbonate and 0.3g acetyl chloride was added and the mixture maintained for a further 5 hours at 120° C. The mixture was filtered, the filtrate concentrated in vacuo and the residue taken up in a small amount of ethyl acetate. The resulting crystals were removed and recrystallised from a small amount of ethyl acetate.

Yield: 0.9 g = 45% of theory
Mp: 244-246° C

Example 6 - Process step f)

6-(6,6-Dimethyl-3,5,6,7-tetrahydropyrrolo[2,1-c][1,2,4]-thiadiazol-3-ylidenimino)-7-fluoro-2H-1,4-benzoxazin-3-(4H)-one

A solution of 0.4 g sodium hydroxide in 5 ml water was added dropwise at 0-3° C to a solution of 1.6 g 2-amino-4,4-dimethyl-1-pyrroline hydrochloride in 20 ml dichloromethane and the mixture stirred for one hour at 0° C. A solution of 1.8g 7-fluoro-6-isothiocyanato-2H-1,4-benzoxazin-3(4H)-one in 200 ml dichloromethane was added dropwise at 0° C at such a rate that the temperature did not go above 5° C. The mixture was stirred for 3 hours at room temperature, cooled to -20° C and a solution of 0.4 ml bromine in 30 ml dichloromethane was added dropwise slowly. The mixture was stirred for a further 3 hours without cooling, the phases separated, the organic phase washed with water, dried over magnesium sulphate and the solvent removed in vacuo. The residue was recrystallised several times from hexane/ethyl acetate.

Yield: 1.23 g = 47% of theory
Mp: 258-259° C

Example 7 - Process step g)

6-(6,6-Dimethyl-3,5,6,7-tetrahydropyrrolo[2,1-c][1,2,4]-thiadiazol-3-ylidenimino)-7-fluoro-4-(2-propynyl)-2H-1,4-benzoxazin-3(4H)-one

A solution of 0.20 g 6-(6,6-dimethyl-3,5,6,7-tetrahydropyrrolo[2,1-c][1,2,4]thiadiazol-3-ylidenimino)-7-fluoro-2H-1,4-benzoxazin-3(4H)-one in 10 ml dimethylformamide was cooled to -5° C. 34 g Sodium hydride was added dropwise and the mixture stirred for one hour at 0° C. 94 mg propargyl chloride was added dropwise, the mixture stirred for 12 hours at room temperature and poured into ice-water. It was extracted several times with ethyl acetate. The combined organic phases were dried over magnesium sulphate and the solvent removed in vacuo. The residue was recrystallised from diisopropyl ether/ethyl acetate.

Yield: 0.20 g = 90% of theory
Mp: 144-146° C

## Claims

1. A process for the preparation of substituted 6-(3,5,6,7-tetrahydropyrrolo[2,1-c][1,2,4]thiadiazol-3-ylidenimino)-7-fluoro-2H-1,4-benzoxazin-3(4H)-ones of general formula I

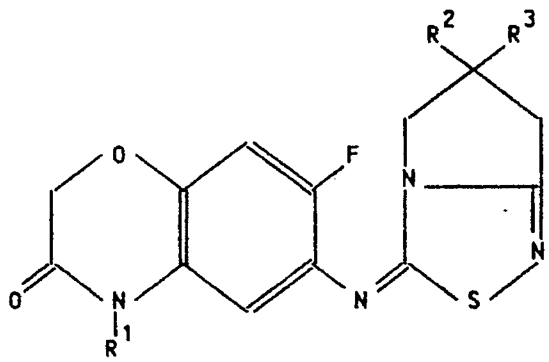

(I)

in which
$R^1$ is $C_1$-$C_6$-alkyl, $C_2$-$C_6$-alkenyl or $C_3$-$C_6$-alkynyl, and
$R^2$ and $R^3$, which may be the same or different, are hydrogen or $C_1$-$C_4$-alkyl, in which

a) a 2-amino-5-fluorophenol of formula II

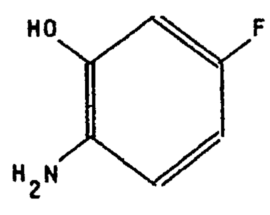

(II)

is reacted with chloracetyl chloride, acetyl chlride or acetic anhydride, in the presence of an acid binding agent, in a suitable solvent.

b) the acetanilide, so formed, of formula IV

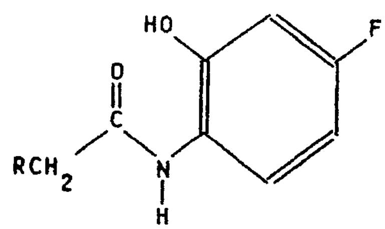

(IV)

in which R is hydrogen or chlorine, is nitrated with nitric acid, an inorganic or organic derivative thereof, optionally in a suitable solvent,

c) the 5-nitroacetanilide, so formed, of formula V

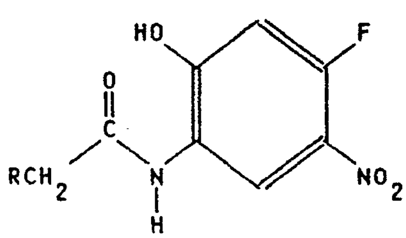

(V)

is hydrogenated with hydrogen, in the presence of a suitable catalyst in an inert solvent, or reduced with a chemical reducing agent in the presence of an inert solvent,

d) the 5-aminoacetanilide, so formed, of formula VI

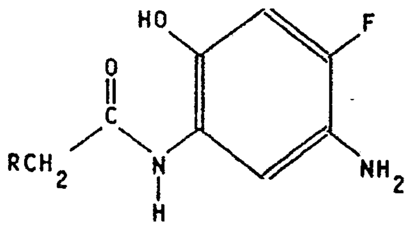

(VI)

is treated with a compound of formula VII

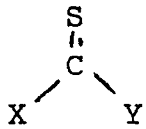

(VII)

in which X is chloro or imidazole and Y is chloro, imidazole or dialkylamino, in a suitable inert solvent, optionally with the addition of an acid binding agent,

e) the 5-isothiocyanatoacetanilide, so formed, of formula VIII

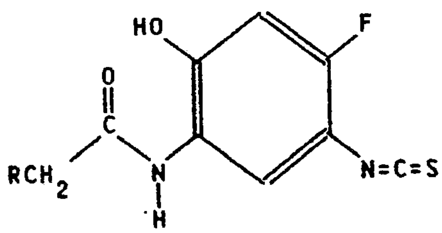

(VIII)

is cyclised in the presence of catalytic to equimolar amounts of an inorganic or organic base,

f) the benzoxazinone isothiocyanate, so formed, of general formula IX

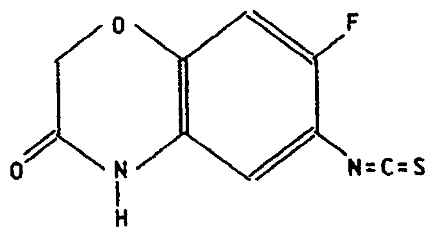

(IX)

is reacted with a compound of general formula X

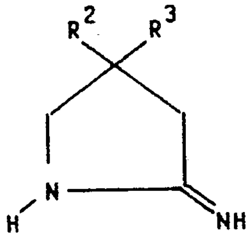

(X)

in which $R^2$ and $R^3$ have the meanings given in general the formula I, in an inert solvent, and the resulting compound cyclised in the presence of an oxidising agentb to the iminothiadiazole ring, and finally

g) the iminothiadiazole, so formed, of general formula XI

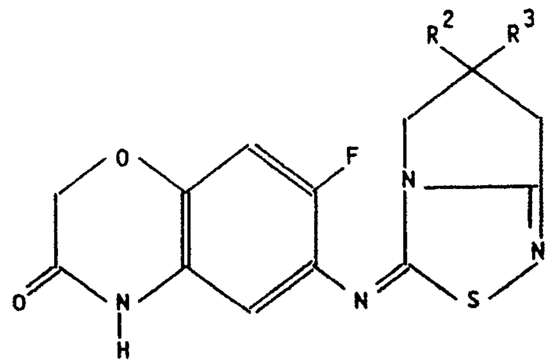

(XI) ,

in which $R^2$ and $R^3$ have the meanings given in general the formula I, is reacted with a compound of general formula XII

$R^1$-Z (XII)

in which $R^1$ has the meaning given in general formula I and Z is chlorine, bromine, iodine, p-toluenesulphonyloxy or methanesulphonyloxy, optionally with the addition of a base in a suitable solvent.

**2.** N-(4-Fluoro-2-hydroxyphenyl)-2-chloracetamides of general formula XIV

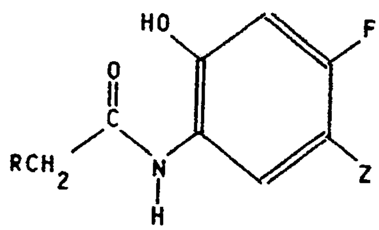

(XIV)

in which R is hydrogen or chlorine, and
Z is hydrogen, nitro, amino or isothiocyanato.